(19) Europäisches Patentamt
European Patent Office
Office européen des brevets

(11) **EP 2 340 889 A1**

(12) **EUROPÄISCHE PATENTANMELDUNG**

(43) Veröffentlichungstag:
**06.07.2011 Patentblatt 2011/27**

(21) Anmeldenummer: **11155485.3**

(22) Anmeldetag: **04.12.2008**

(51) Int Cl.:
*B01J 39/20* (2006.01)    *B01J 39/26* (2006.01)
*C08F 8/36* (2006.01)    *C02F 1/42* (2006.01)
*B01J 31/10* (2006.01)    *C08F 257/02* (2006.01)

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **18.12.2007 DE 102007060790**

(62) Dokumentnummer(n) der früheren Anmeldung(en) nach Art. 76 EPÜ:
**08170635.0 / 2 077 158**

(71) Anmelder: **LANXESS Deutschland GmbH 51369 Leverkusen (DE)**

(72) Erfinder:
• **Vanhoorne, Pierre**
  **40789, Monheim (DE)**
• **Wedemeyer, Hans-Jürgen**
  **51371, Leverkusen (DE)**

Bemerkungen:
Diese Anmeldung ist am 22-02-2011 als Teilanmeldung zu der unter INID-Code 62 erwähnten Anmeldung eingereicht worden.

(54) **Verfahren zur Herstellung von Kationenaustauschern**

(57) Starksaure Kationenaustauscher mit hoher mechanischen, osmotischen und Oxidations-Stabilität lassen sich durch Sulfonierung von Perlpolymerisaten aus einem oder mehreren vinylaromatischen Monomer(en), einem oder mehreren Vernetzer(n) und 0,2 bis 20 Gew.-% einer oder mehrerer Vinylether und/oder Vinylester herstellen.

EP 2 340 889 A1

**Beschreibung**

Die Erfindung betrifft ein Verfahren zur Herstellung von starksauren Kationenaustauschern mit hoher mechanischer, osmotischer und Oxidations-Stabilität durch Sulfonierung von Perlpolymerisaten aus einem oder mehreren vinylaromatischen Monomer(en), einem oder mehreren Vernetzer(n) und einem oder mehreren Ester(n) des Vinylalkohols.

Stark saure Kationenaustauscher lassen sich durch Funktionalisieren von vernetzten Styrol-Perlpolymerisaten erhalten. Bei der Funktionalisierung werden durch Reaktion aromatischer Einheiten des Polymergerüstes mit einem Sulfonierungsmittel, wie z.B. Schwefelsäure, kovalent gebundene Sulfonsäuregruppen erzeugt.

Ein Problem der bekannten starksauren Kationenaustauscher besteht in ihrer nicht immer ausreichenden Stabilität bei Beanspruchung. So können Kationenaustauscherperlen durch mechanische oder osmotische Kräfte zerbrechen. Für alle Anwendungen von Kationenaustauschern gilt, dass die in Perlform vorliegenden Austauscher ihren Habitus behalten müssen und nicht während der Anwendung teilweise oder auch gänzlich abgebaut werden oder in Bruchstücke zerfallen dürfen. Bruchstücke und Perlpolymerisatsplitter können während der Reinigung in die zu reinigenden Lösungen gelangen und diese selbst verunreinigen. Ferner ist das Vorhandensein von geschädigten Perlpolymerisaten für die Funktionsweise der in Säulenverfahren eingesetzten Kationenaustauscher selbst ungünstig. Splitter führen zu einem erhöhten Druckverlust des Säulensystems und vermindern damit den Durchsatz der zu reinigenden Flüssigkeit durch die Säule.

Ein weiteres Problem der bekannten starksauren Kationenaustauscher ist deren Neigung, in der Anwendung durch die Einwirkung verschiedenster im Wasser gelöster Oxidationsmittel (Luftsauerstoff, Wasserstoffperoxid, Vanadylsalze, Chromate) aus Wasser sulfonierte, wasserlösliche Bruchstücke abzugeben. Dieses Phänomen, dem Fachmann im Allgemeinen unter dem Begriff Leaching bekannt, führt zur Anreicherung sulfonierter organischer Bestandteile im zu behandelnden Wasser, was zu verschiedenen Problemen in den nachgeschalteten Anlagen führen kann, die auf die Zufuhr von voll entsalztem Wasser angewiesen sind. So führen die wasserlöslichen Bruchstücke z. B. im Kühlkreislauf von Kraftwerken zu Korrosionsproblemen, in der Elektronikindustrie zu Defekten in den hergestellten Mikrochips, in Erodiermaschinen zum Versagen der Anlage wegen zu hoher elektrischer Leitfähigkeit des Wassers.

Es hat nicht an Versuchen gefehlt, starksaure Kationenaustauscher bereitzustellen, die eine verbesserte mechanische, osmotische und/oder eine verbesserte Oxidationsstabilität aufweisen.

So kann die mechanische und osmotische Stabilität starksaurer Kationenaustauscher durch einen zweistufigen Aufbau der Perlpolymerisate, in einem so genannten Seed-Feed-Verfahren, erhöht werden. Solche Verfahren werden zum Beispiel in EP 0 101 943 A2, EP-A 1 000 659 und DE- A 10 0 122 896 beschrieben. Die zweistufig hergestellten Kationenaustauscher zeigen eine hervorragende mechanische Stabilität, geben aber deutlich mehr sulfonierte Abbauprodukte an das behandelte Wasser ab, als die einstufig hergestellten Harze.

Auch der Einbau kleiner Mengen Acrylatmonomere in das Styrol-Divinylbenzol-Copolymer führt zu einer erhöhten mechanischen Stabilität, wie in US 4 500 652 bzw. DE-A 3 230 559 beschrieben. Die Anwesenheit von Acrylsäureeinheiten in der Polymerstruktur führt allerdings zu einer höheren Oxidationsanfälligkeit der Harze.

Bachmann et al. beschreiben in EP-A 868 444 ein Verfahren zur Herstellung von mechanisch und osmotisch stabilen Kationenaustauschern durch Sulfonierung ohne Zusatz von chlorierten Quellmitteln bei Temperaturen zwischen 125 und 180 °C. Durch den Verzicht auf das chlorierte Quellmittel bei der Sulfonierung wird aber die Oxidationsstabilität der Harze nicht verbessert.

Die Oxidationsstabilität der starksauren Kationenaustauscher kann durch Zugabe von Antioxidantien erhöht werden, wie es zum Beispiel in EP-A 0 366 258 beschrieben wird. Diese Antioxidantien werden langsam aus dem Harz herausgewaschen, was zur Abgabe von organischen Bestandteilen an das behandelte Wasser führt. Darüber hinaus werden sie nach einer relativ kurzen Zeit in der Anwendung aufgebraucht. Danach zeigt das mit Antioxidantien behandelte Harz dieselbe Oxidationsempfindlichkeit wie ein herkömmlicher starksaurer Kationenaustauscher.

Die Oxidationsstabilität der starksauren Kationenaustauscher kann auch durch Erhöhung der Vernetzungsdichte des Perlpolymerisates verbessert werden. Durch den Einbau von größeren Mengen an Vernetzer werden die Polymerisate aber spröder, was zu einer deutlichen Reduzierung der mechanischen Stabilität der Perlen führt. Außerdem nimmt die Kinetik des Kationenaustauschs mit zunehmender Vernetzungsdichte deutlich ab, was in vielen Anwendungen zu unzureichenden Aufnahmekapazitäten führt.

Es besteht also weiterhin ein Bedarf an Kationenaustauschern mit schneller Austauschkinetik, hoher mechanischer und osmotischer Stabilität und gleichzeitig hoher Oxidationsstabilität.

Die Aufgabe der vorliegenden Erfindung ist daher die Bereitstellung eines einfachen, robusten und wirtschaftlichen Verfahrens zur Herstellung von Kationenaustauschern mit schneller Austauschkinetik, hoher mechanischer und osmotischer Stabilität und hoher Oxidationsstabilität.

Lösung der Aufgabe und somit Gegenstand der vorliegenden Erfindung ist ein Verfahren zur Herstellung von starksauren Kationenaustauschern durch Sulfonierung vernetzter Perlpolymerisate aus vinylaromatischen Monomeren, dadurch gekennzeichnet, dass die Perlpolymerisate 0,2 bis 20 Gew.-% Vinylester als Comonomer(e) enthalten.

Überraschenderweise weisen Kationenaustauscher, die man durch das erfindungsgemäße Verfahren erhält, eine gegenüber dem Stand der Technik deutlich höhere Oxidations-Stabilität bei gleich bleibenden oder höheren mechanische und osmotische Stabilitäten auf. Es wurde zudem überraschend gefunden, dass die Verbesserung der Oxidations-Stabilität der aus den erfindungsgemäßen Perlpolymerisaten gewonnenen starksauren Kationenaustauschern einzig und alleine auf den Einbau des Comonomers im Perlpolymerisat zurückzuführen ist, unabhängig davon, auf welcher Weise und zu welchem Zeitpunkt bei der Bildung des Perlpolymerisates das Comonomer zugegeben und einpolymerisiert wird.

Zur Klarstellung sei angemerkt, dass vom Rahmen der Erfindung alle nachfolgend aufgeführten, allgemeinen oder in Vorzugsbereichen genannten Definitionen und Parameter in beliebigen Kombinationen umfasst sind.

Erfindungsgemäß geeignete vernetzte Perlpolymerisate sind Copolymerisate aus mindestens einem monoethylenisch ungesättigten aromatischen Monomer, mindestens einem Vernetzer und mindestens einem Vinylester.

Als monoethylenisch ungesättigte aromatische (=vinylaromatische) Monomere werden bevorzugt Styrol, $\alpha$-Methylstyrol, Vinyltoluol, Ethylstyrol, t-Butylstyrol, Chlorstyrol, Bromstyrol, Chlormethylstyrol oder Vinylnaphthalin eingesetzt. Gut geeignet sind auch Mischungen dieser Monomere. Besonders bevorzugt sind Styrol und Vinyltoluol.

Den Monomeren werden Vernetzer beigemischt. Vernetzer sind im Allgemeinen multiethylenisch ungesättigte Verbindungen, bevorzugt Divinylbenzol, Divinyltoluol, Trivinylbenzol, Octadien oder Triallylcyanurat. Besonders bevorzugt sind die vinylaromatischen Vernetzer Divinylbenzol und Trivinylbenzol. Ganz besonders bevorzugt ist Divinylbenzol. Zur Herstellung der Perlpolymerisate können technische Qualitäten von Divinylbenzol eingesetzt werden, die neben den Isomeren des Divinylbenzols übliche Nebenprodukte wie Ethylvinylbenzol enthalten. Erfindungsgemäß sind technische Qualitäten mit Divinylbenzolgehalten von 55 bis 85 Gew. % besonders gut geeignet.

Die Vernetzer können alleine oder als Gemisch verschiedener Vernetzer eingesetzt werden. Die Gesamtmenge einzusetzender Vernetzer beträgt in der Regel 0,1 bis 80 Gew.-%, bevorzugt 0,5 bis 60 Gew.-%, besonders bevorzugt 1 bis 40 Gew.-%, bezogen auf die Summe der ethylenisch ungesättigten Verbindungen.

Als Comonomer(e) werden Vinylester eingesetzt.

Unter Vinylether im Sinne der vorliegenden Erfindung werden die Ether des Vinylalkohols und des Isopropenylalkohols verstanden. Vinylether im Sinne der vorliegenden Erfindung können eine oder mehrere Vinyl- bzw. Isopropenylalkoholeinheiten enthalten. Bevorzugt werden Alkyl- und Hydroxyalkylether mit 1 bis 18 C-Atomen sowie Ether mit Kondensationsprodukten des Ethylenglykols. Besonders bevorzugt werden Methylvinylether, Ethylvinylether, Ethylenglykolmonovinylether, Ethylenglykoldivinylether, Diethylenglykolmonovinylether, Diethylenglykoldivinylether, Butandiolmonovinylether, Butandioldivinylether, Methylisopropenylether oder Ethylisopropenylether. Ganz besonders bevorzugt werden Ethylenglykoldivinylether, Diethylenglykoldivinylether und Butandioldivinylether eingesetzt.

Unter Vinylester im Sinne der vorliegenden Erfindung werden die Ester des Vinylalkohols und des Isopropenylalkohols verstanden. Vinylester im Sinne der vorliegenden Erfindung können eine oder mehrere Vinyl- bzw. Isopropenylalkoholeinheiten enthalten. Bevorzugt werden Ester von Carbonsäuren mit 1 bis 18 C-Atomen. Besonders bevorzugt werden Vinylformiat, Vinylacetat, Vinylpropionat, Vinylbutyrat, Vinylvalerat, Vinylhexanoat, Vinyloctoat, Vinyldecanoat, Vinyllaurat, Vinylmyristat, Vinyloleat, Vinylpalmitat, Vinylbenzoat, Divinylphthalat oder Isopropenylacetat. Ganz besonders bevorzugt werden Vinylacetat und Isopropenylacetat eingesetzt.

Es können auch Gemische von Vinylestern oder Gemische von Vinylethern mit Vinylestern eingesetzt werden.

Das Comonomer wird in Mengen von 0,2 bis 20 Gew.-%, bezogen auf die Summe von vinylaromatischen Monomeren und Vernetzern, eingesetzt. Bevorzugt werden Mengen von 0,5 bis 15 Gew.-%, besonders bevorzugt von 1 bis 10 Gew.-% verwendet. Werden Gemische von Vinylethern und/oder Vinylestern eingesetzt, beziehen sich die Mengenangaben auf die Summe aller Comonomere.

Das Comonomer kann in einer bevorzugten Ausführungsvariante der vorliegenden Erfindung dem Monomergemisch vor Einsetzen der Polymerisation zugesetzt werden. Es kann aber auch im Laufe der Polymerisation, bevorzugt bei einem Polymerisationsumsatz zwischen 10 und 90 %, besonders bevorzugt zwischen 15 und 80 %, zudosiert werden.

In einer weiteren bevorzugten Ausführungsvariante der vorliegenden Erfindung wird das Comonomer im Laufe der Polymerisation zusammen mit einem wasserlöslichen Initiator der wässrigen Phase zugegeben. Geeignete wasserlösliche Initiatoren in dieser bevorzugten Ausführungsvariante sind Verbindungen, die bei Temperaturerhöhung freie Radikale bilden. Bevorzugt werden Peroxodisulfate, besonders bevorzugt Kaliumperoxodisulfat, Natriumperoxodisulfat und Ammoniumperoxodisulfat, wasserlösliche Azoverbindungen, besonders bevorzugt 2,2'-Azobis(2-amidinopropan) hydrochlorid, 2,2'-Azobis[N-(2-carboxyethyl)-2-methylpropionamidin]tetrahydrat, 2,2'-Azobis[N,N'-dimethyleneisobutyramidin], 4,4'-Azobis(4-cyanovaleriansäure) sowie Hydroperoxide, besonders bevorzugt t-Butylhydroperoxid und Cumylhydroperoxid.

Das Comonomer kann aber auch nach Vollendung der Polymerisation des Perlpolymerisats zugegeben werden und in einem separaten Polymerisationsschritt polymerisiert werden.

In einer bevorzugten Ausführungsform der vorliegenden Erfindung können den Monomeren auch Porenbildner, so genannte Porogene, zugesetzt werden. Die Porogene dienen der Bildung einer Porenstruktur im nicht funktionellen Perlpolymerisat. Als Porogene werden bevorzugt organische Verdünnungsmittel eingesetzt. Besonders bevorzugt werden solche organischen Verdünnungsmittel, die sich zu weniger als 10 Gew.-%, vorzugsweise weniger als 1 Gew.-% in Wasser lösen, eingesetzt. Insbesondere geeignete Porogene sind Toluol, Ethylbenzol, Xylol, Cyclohexan, Oktan, Isooktan, Decan, Dodekan, Isododekan, Methylisobutylketon, Ethylacetat, Butylacetat, Dibutylphtalat, n-Butanol, 4-Methyl-2-pentanol oder n-Octanol. Ganz besonders bevorzugt sind Toluol, Cyclohexan, Isooktan, Isododekan, 4-Methyl-2-pentanol oder Methylisobutylketon. Als Porogene können auch unvernetzte, lineare oder verzweigte Polymere eingesetzt werden, wie z.B. Polystyrol und Polymethylmethacrylat. Geeignet sind auch Gemische von verschiedenen Porogenen.

Das Porogen wird üblicherweise in Mengen von 10 bis 70 Gew.-%, bevorzugt 25 bis 65 Gew.-%, jeweils bezogen auf die Summe der ethylenisch ungesättigten Verbindungen, eingesetzt.

Zur Herstellung der vernetzten Perlpolymerisate werden die oben genannten Monomere in einer weiteren bevorzugten Ausführungsform der vorliegenden Erfindung in Anwesenheit eines Dispergierhilfsmittels unter Verwendung eines Initiators in wässriger Suspension polymerisiert.

Als Dispergierhilfsmittel werden bevorzugt natürliche und synthetische wasserlösliche Polymere eingesetzt. Besonders bevorzugt werden Gelatine, Stärke, Cellulosederivate, Polyvinylalkohol, Polyvinylpyrrolidon, Polyacrylsäure, Polymethacrylsäure oder Copolymerisate aus (Meth)acrylsäure und (Meth)acrylsäureestern eingesetzt. Ganz besonders bevorzugt werden Gelatine oder Cellulosederivate, insbesondere Celluloseester und Celluloseether, wie Carboxymethylcellulose, Methylcellulose, Hydroxyethylcellulose oder Methylhydroxyethylcellulose eingesetzt. Die Einsatzmenge der Dispergierhilfsmittel beträgt im Allgemeinen 0.05 bis 1 %, vorzugsweise 0.1 bis 0.5 %, bezogen auf die Wasserphase.

In einer weiteren bevorzugten Ausführungsform der vorliegenden Erfindung werden im Monomergemisch Initiatoren eingesetzt. Als Monomergemisch wird in der vorliegenden Erfindung das Gemisch aus vinylaromatischen Monomeren, Vernetzer(n), Comonomer(en) und gegebenenfalls Porogen(en) bezeichnet. Geeignete Initiatoren sind Verbindungen, die bei Temperaturerhöhung freie Radikale bilden und sich im Monomergemisch lösen. Bevorzugt werden Peroxyverbindungen, besonders bevorzugt Dibenzoylperoxid, Dilaurylperoxid, Bis-(p-chlorbenzoyl)peroxid, Dicyclohexylperoxydicarbonat oder tert.-Amylperoxy-2-ethylhexan sowie Azoverbindungen, besonders bevorzugt 2,2'-Azobis(isobutyronitril) oder 2,2'-Azobis(2-methylisobutyronitril) oder auch aliphatische Peroxyester, bevorzugt tert.-Butylperoxyacetat, tert.-Butylperoxyisobutyrat, tert.-Butylperoxypivalat, tert.-Butylperoxyoctoat, tert.-Butylperoxy-2-ethylhexanoat, tert.-Butylperoxyneodecanoat, tert.-Amylperoxypivalat, tert.-Amylperoxyoctoat, tert.-Amylperoxy-2-ethylhexanoat, tert.-Amylperoxyneodecanoat, 2,5-Bis(2-ethylhexanoylperoxy)-2,5-dimethylhexan, 2,5-Dipivaloyl-2,5-dimethylhexan, 2,5-Bis(2-neodecanoylperoxy)-2,5-dimethylhexan, Di-tert.-butylperoxyazelat oder Di-tert.-amylperoxyazelat eingesetzt.

Die im Monomergemisch löslichen Initiatoren werden im Allgemeinen in Mengen von 0,05 bis 6,0 Gew.-%, vorzugsweise 0,1 bis 5,0 Gew.-%, besonders bevorzugt 0,2 bis 2 Gew.-%, bezogen auf die Summe der ethylenisch ungesättigten Verbindungen, angewendet.

Die Wasserphase kann ein Puffersystem enthalten, welches den pH-Wert der Wasserphase auf einen Wert zwischen 12 und 3, vorzugsweise zwischen 10 und 4 einstellt. Besonders gut geeignete Puffersysteme enthalten Phosphat-; Acetat-, Citrat- oder Boratsalze.

Es kann vorteilhaft sein, einen in der wässrigen Phase gelösten Inhibitor einzusetzen. Als Inhibitoren kommen sowohl anorganische als auch organische Stoffe in Frage. Beispiele für anorganische Inhibitoren sind Stickstoffverbindungen wie Hydroxylamin, Hydrazin, Natriumnitrit oder Kaliumnitrit. Beispiele für organische Inhibitoren sind phenolische Verbindungen wie Hydrochinon, Hydrochinonmonomethylether, Resorcin, Brenzkatechin, tert.-Butylbrenzkatechin, Kondensationsprodukte aus Phenolen mit Aldehyden. Weitere organische Inhibitoren sind stickstoffhaltige Verbindungen wie z.B. Diethylhydroxylamin und Isopropylhydroxylamin. Resorcin wird als Inhibitor bevorzugt. Die Konzentration des Inhibitors beträgt 5 - 1000 ppm, vorzugsweise 10 - 500 ppm, besonders bevorzugt 20 - 250 ppm, bezogen auf die wässrige Phase.

Die organische Phase kann als Tröpfchen durch Rühren bzw. durch Verdüsung (Jetting) in die wässrige Phase dispergiert werden. Unter organischer Phase wird die Mischung aus Monomer(en) und Vernetzter(n) sowie gegebenenfalls zusätzlich Porogen(en) und / oder Initiator(en) verstanden. Bei der klassischen Dispersionspolymerisation werden die organischen Tröpfchen durch Verrühren erzeugt. Im 4 Liter Maßstab werden typischerweise Rührerdrehzahlen von 250 bis 400 UpM verwendet. Werden die Tröpfchen durch Verdüsung erzeugt, empfiehlt es sich, zur Erhaltung des einheitlichen Tröpfchendurchmessers die organischen Tröpfchen zu verkapseln. Verfahren zur Mikroverkapselung von verdüsten organischen Tröpfchen werden beispielsweise in EP-A 0 046 535 beschrieben, deren Inhalt in Bezug auf die Mikroverkapselung von der vorliegenden Anmeldung mit umfasst wird.

Die mittlere Teilchengröße der gegebenenfalls verkapselten Monomertröpfchen beträgt 10-1000 $\mu$m, vorzugsweise 100 - 1000 $\mu$m.

Das Verhältnis der organischen Phase zur wässrigen Phase beträgt in der Regel 1:20 bis 1:0,6, bevorzugt 1:10 bis 1:1, besonders bevorzugt 1:5 bis 1:1,2.

Die organische Phase kann aber auch, gemäß EP-A 0 617 714 deren Lehre von der vorliegenden Anmeldung mit umfasst wird, im so genannten Seed-Feed-Verfahren zu einer Suspension von Saatpolymerisaten, die die organische Phase aufnehmen, zugegeben werden. Die mittlere Teilchengröße der mit der organischen Phase gequollenen Saatp-

olymerisate beträgt 5-1200 μm, vorzugsweise 20 - 1000 μm. Das Verhältnis der Summe organische Phase + Saatpolymerisat zur wässrigen Phase beträgt in der Regel 1:20 bis 1:0,6, bevorzugt 1:10 bis 1:1, besonders bevorzugt 1:5 bis 1:1,2.

Die Polymerisation der Monomere und Comonomere wird bei erhöhter Temperatur durchgeführt. Die Polymerisationstemperatur richtet sich nach der Zerfallstemperatur des Initiators und liegt typischerweise im Bereich von 50 bis 150°C, vorzugsweise 60 bis 130°C. Die Polymerisationsdauer liegt bei 30 Minuten bis 24 Stunden, bevorzugt 2 bis 15 Stunden.

Am Ende der Polymerisation werden die vernetzten Perlpolymerisate von der wässrigen Phase abgetrennt, bevorzugt auf einer Nutsche, und gegebenenfalls getrocknet.

Die Umsetzung der vernetzten Perlpolymerisate zu Kationenaustauschern erfolgt durch Sulfonierung. Als Sulfonierungsmittel kommen in Frage Schwefelsäure, Chlorsulfonsäure und Schwefeltrioxid. Bevorzugt wird Schwefelsäure eingesetzt.

Die Schwefelsäure wird bevorzugt in einer Konzentration von 80 bis 120 %, besonders bevorzugt 85 bis 105 %, ganz besonders bevorzugt 88 bis 99 % eingesetzt. Für die Schwefelsäure bedeuten in der vorliegenden Erfindung Konzentrationsangaben über 100% Lösungen von Schwefeltrioxid ($SO_3$) in 100%iger Schwefelsäure. So ist eine Schwefelsäurekonzentration von 120% als 20 Gew.-%ige Lösung von $SO_3$ in 100%iger Schwefelsäure zu verstehen.

Es ist vorteilhaft, die notwendige Säurekonzentration durch Mischen von Schwefelsäure einer höheren und einer niedrigeren Konzentration einzustellen, wobei als Schwefelsäure mit niedrigerer Konzentration zurückgewonnene Schwefelsäure aus früheren Sulfonierungsreaktionen eingesetzt werden kann. Das Mischen der Schwefelsäure kann im Sulfonierungsreaktor in Anwesenheit des zu sulfonierenden Perlpolymerisats erfolgen, so dass die auftretende Mischungswärme zu einer Temperaturerhöhung des Reaktionsgemisches führt.

Das Verhältnis von Schwefelsäure zu Perlpolymerisat beträgt 2,0 bis 6 ml/g, vorzugsweise 2,5 bis 5 ml/g, besonders bevorzugt 2,6 bis 4,2 ml/g.

Falls gewünscht, kann bei der Sulfonierung ein Quellungsmittel, bevorzugt Chlorbenzol, Dichlorethan, Dichlorpropan oder Methylenchlorid angewendet werden. Das Quellungsmittel wird bevorzugt in Mengen von 0,1 bis 1 ml pro Gramm trockenes Perlpolymerisat, besonders bevorzugt 0,2 bis 0,5 ml pro Gramm trockenes Perlpolymerisat, eingesetzt. Das Quellungsmittel wird bevorzugt dem in Schwefelsäure vorgelegten Perlpolymerisat vor dem Einsetzen der Sulfonierungsreaktion zugegeben.

Die Temperatur bei der Sulfonierung liegt im Allgemeinen bei 50 - 200°C, bevorzugt bei 80 - 160 °C, besonders bevorzugt bei 90 - 140 °C. Es kann vorteilhaft sein, bei der Sulfonierung ein Temperaturprogramm anzuwenden, in dem die Sulfonierung in einem ersten Reaktionsschritt bei einer ersten Temperatur begonnen wird und in einem zweiten Reaktionsschritt bei einer höheren Temperatur fortgeführt wird.

Bei der Sulfonierung wird das Reaktionsgemisch gerührt. Dabei können verschiedene Rührertypen, wie Blatt-, Anker-, Gitter- oder Turbinenrührer eingesetzt werden.

Die Dauer der Sulfonierungsreaktion beträgt im Allgemeinen mehrere Stunden, bevorzugt zwischen 1 und 24 h, besonders bevorzugt zwischen 2 und 16 h, ganz besonders bevorzugt zwischen 3 und 12 h.

Nach der Sulfonierung wird das Reaktionsgemisch aus Sulfonierungsprodukt und Restsäure auf Raumtemperatur abgekühlt und zunächst mit Schwefelsäuren abnehmender Konzentrationen und dann mit Wasser verdünnt.

Falls gewünscht kann der erfindungsgemäß erhaltene Kationenaustauscher in der H-Form zur Reinigung mit entionisiertem Wasser bei Temperaturen von 70 - 180 °C, vorzugsweise von 105 - 130 °C behandelt werden.

Für viele Anwendungen ist es günstig, den Kationenaustauscher von der sauren Form in die Natrium-Form zu überführen. Diese Umladung erfolgt mit Natronlauge einer Konzentration von 2 - 60 Gew. %, vorzugsweise 4 -10 Gew. % oder mit wässrigen Natriumchloridlösungen, die 1 - 25 gew. % ig, bevorzugt 4 - 10 gew. % an Natriumchlorid sind.

Nach der Umladung können die Kationenaustauscher zur weiteren Reinigung mit entionisiertem Wasser oder wässrigen Salzlösungen, beispielsweise mit Natriumchlorid- oder Natriumsulfatlösungen, behandelt werden. Dabei wurde gefunden, dass die Behandlung bei 70 - 150 °C, vorzugsweise 120- 135° besonders effektiv ist und keine Verringerung der Kapazität des Kationenaustauschers bewirkt.

Die erfindungsgemäßen, starksauren Kationenaustauscher können Poren enthalten. Poröse erfindungsgemäßen starksauren Kationenaustauscher können mikroporös, mesoporös und/oder makroporös sein. Zur Definition der Begriffe "gelförmig", "porös", "mikroporös", "mesoporös" und "makroporös" für Polymere wird auf Pure Appl. Chem., Vol. 76, No. 4, S. 889-906, 2004 (IUPAC recommendations 2003), insb. S. 900 § 3.9 und S. 902-903 § 3.23, verwiesen.

Die erfindungsgemäßen, starksauren Kationenaustauscher weisen eine mittlere Teilchengröße D zwischen 30 μm und 1000 μm auf, bevorzugt zwischen 100 und 800 μm. Zur Bestimmung der mittleren Teilchengröße und der Teilchengrößenverteilung sind übliche Methoden, wie Siebanalyse oder Bildanalyse geeignet. Als mittlere Teilchengröße D wird im Sinne der vorliegenden Erfindung der 50%-Wert (Ø (50)) der Volumenverteilung verstanden. Der 50%-Wert (Ø (50) der Volumenverteilung gibt den Durchmesser an, der von 50 Vol.-% der Teilchen unterschritten wird.

In einer bevorzugten Ausführung der vorliegenden Erfindung werden monodisperse starksaure Kationenaustauscher hergestellt. Monodisperse Teilchengrößenverteilungen im Sinne der vorliegenden Erfindung weisen einen Volumenanteil an Teilchen zwischen 0,9 D und 1,1 D von mindestens 75 Vol.-%, bevorzugt mindestens 85 Vol.-%, besonders bevorzugt mindestens 90 Vol.-% auf.

Die vorliegende Erfindung betrifft aber auch ein Verfahren zur Herstellung starksaurer Kationenaustauscher dadurch gekennzeichnet, dass man:

a) monodisperse oder heterodisperse Perlpolymerisate aus vinylaromatischen Monomeren, Vernetzern und 0,2 bis 20 Gew.-% Vinylester durch Suspensionspolymerisation herstellt und

b) diese Perlpolymerisate durch Einwirkung von Schwefelsäure, Schwefeltrioxid und/oder Chlorsulfonsäure in starksaure Kationenaustauscher überführt.

Die nach dem erfindungsgemäßen Verfahren erhaltenen starksauren Kationenaustauscher zeichnen sich durch eine besonders hohe mechanische, osmotische und Oxidations-Stabilität aus. Sie zeigen auch nach längerem Gebrauch und vielfacher Regeneration kaum Defekte an den Ionenaustauscherkugeln.

Für die erfindungsgemäßen starksauren Kationenaustauscher gibt es eine Vielzahl unterschiedlicher Anwendungen. So können sie beispielsweise bei der Trinkwasseraufbereitung, bei der Herstellung von Kraftwerkswasser und Reinstwasser (notwendig bei der Mikrochip-Herstellung für die Computerindustrie), zur chromatographischen Trennung von Glucose und Fructose und als Katalysatoren für verschiedene chemische Reaktionen (wie z. B. bei der Bisphenol-A-Herstellung aus Phenol und Aceton) eingesetzt werden.

Untersuchungsmethoden:

**Volumenbezogene Totale Kapazität**

20 ml Austauscher werden auf dem Stampfvolumeter unter VE-Wasser (vollentsaltzes Wasser) eingerüttelt. In einem 200 ml-Becherglas werden diese 20 ml Austauscher, 5 g NaCl p.a und 50 ml Natronlauge c(NaOH)= 1 Mol/L zusammengegeben und mit Salzsäure c(HCl)= 1,0 Mol/L bis zu pH=4,3 titriert.

Die Totale Kapazität des Kationenaustauschers wird wie folgt berechnet:

$$TK = \frac{\text{Verbrauch HCl c (HCl)} = 1{,}0\,\text{Mol/l}}{20}$$

**Originalstabilität: Anzahl perfekter Perlen nach Herstellung**

100 Perlen werden unter dem Mikroskop betrachtet. Ermittelt wird die Anzahl der Perlen, die Risse tragen oder Absplitterungen zeigen. Die Anzahl perfekter Perlen ergibt sich aus der Differenz der Anzahl beschädigter Perlen zu 100.

**Bestimmung der osmotischen Stabilität von Kationenaustauschern durch Quellungsstabilität.**

Bei der Quellungsstabilitäts-Prüfung werden die Austauscher im Filterrohr wechselweise mit Salzsäure w(HCl) =6% und Natronlauge w(NaOH)=4% behandelt. Zwischendurch wird jeweils mit VE-Wasser behandelt.

Zur Prüfung werden 25 ml unter VE-Wasser eingerüttelter Austauscher in ein Filterrohr eingebaut.

Dann wird das Harz 5 Minuten mit VE-Wasser rückgespült. Die Rückspülgeschwindigkeit wird so reguliert, dass das Harz über die gesamte Filterrohrlänge verteilt ist.

Nach beendetem Rückspülen werden 40 Arbeitsspiele durchgeführt. Ein Arbeitsspiel umfasst 4 Takte von je 10 Minuten Beladen und Regenerieren und 2 mal je 5 Minuten Rückspülen. Säure und Lauge laufen durch Kapillaren mit 500 ml pro Takt über die Austauscher.

Nach beendeter Prüfung spült man den Austauscher aus dem Filterrohr, saugt das Wasser mit dem Siebrohr ab und mischt gut durch.

Der Austauscher wird dann unter dem Mikroskop auf %-Anteil der ganzen Perlen, der gerissenen Perlen und Splitter wie bei der Bestimmung der Originalstabilität ausgezählt.

**Bestimmung der Oxidations-Stabilität von Kationenaustauschern durch Rotfärbungstest.**

Es werden 750 ml Harz eingerüttelt und 4 Stunden mit einer Geschwindigkeit von 15 L/h mit UPW Wasser im Gleichstrom ausgewaschen. UPW-Wasser wird definiert als Wasser mit einer Leitfähigkeit < 17,8 MOhm cm und einem Gehalt an organischem Material (TOC Gehalt) < 2,0 ppb. Nach dem Auswaschen werden die 750 ml Harz auf einer Glasnutsche 5 Minuten abgesaugt. Zur Leifähigkeit von UPW siehe auch 1998 Semiconductor Pure Water and Chemicals Conference, March 2-5, 1998, Advances in Resistivity Instrumentation for UPW Systems of the Future, Anthony C. Bevilacqua unter:

http://www.gatewayequipment.com/whitepapers/resistivity_instru_futureUPWsystems.pdf.

Es werden 50 g des ausgewaschenen und abgesaugten Kationenaustauschers in eine Glasflasche überführt. Die verschlossene Glasflasche wird bei Raumtemperatur für 4 Wochen bei Tageslicht gelagert. Am Ende der Lagerzeit wird das Harz mit 100 g UPW Wasser versetzt und 10 Minuten mit 100 U/min geschüttelt. Anschließend wird die Probe abfiltriert und das Eluat nach folgenden Methoden geprüft: pH-Wert, Extinktion bei 225 nm (1 cm Küvette) und visuelle Beurteilung der Rotfärbung, wobei die Note 0 ein völlig farbloses Eluat und die Note 4 ein tiefrot gefärbtes Eluat kennzeichnet.

VE-Wasser oder voll entsalztes Wasser im Sinne der vorliegenden Erfindung ist gekennzeichnet, indem es eine Leitfähigkeit von 0,1 bis 10 $\mu$S besitzt, wobei der Gehalt an gelösten oder ungelösten Metallionen nicht größer als 1 ppm, bevorzugt nicht größer als 0,5 ppm für Fe, Co, Ni, Mo, Cr, Cu als individuelle Komponenten ist und nicht größer als 10 ppm, bevorzugt nicht größer als 1 ppm für die Summe der genannten Metalle ist.

## Beispiele

### Beispiel 1

*Herstellung eines monodispersen Saatperlpolymerisates auf der Basis von Styrol, Divinylbenzol und Ethylstyrol.*

In einem 4 1 Glasreaktor wurden 1020 g VE Wasser vorgelegt und eine Lösung aus 3,2 g Gelatine, 4,8 g di-Natriumhydrogenphosphatdodekahydrat und 0,24 g Resorcin in 86 g entionisiertem Wasser hinzugefüllt und durchmischt. Die Mischung wurde auf 25 °C temperiert. Unter Rühren wurde anschließend 1182 g durch Verdüsung (Jetting) gewonnene, mikroverkapselten Monomertröpfchen mit enger Teilchengrössenverteilung, enthaltend 4,5 Gew.-% Divinylbenzol, 1,12 Gew.-% Ethylstyrol, 0,36 Gew.-% tert.-Butylperoxy-2-ethylhexanoat und 94,02 Gew.-% Styrol, gegeben, wobei die Mikrokapsel aus einem mit Formaldehyd gehärteten Komplexkoazervat aus Gelatine und einem Copolymer aus Acrylamid und Acrylsäure bestanden, und 1182 g wässriger Phase mit einem pH-Wert von 12 zugesetzt.
Der Ansatz wurde unter Rühren durch Temperaturerhöhung nach einem Temperaturprogramm bei 25°C beginnend und bei 95°C endend auspolymerisiert. Der Ansatz wurde abgekühlt, über ein 315 $\mu$m-Sieb gewaschen und anschließend im Vakuum bei 80°C getrocknet. Man erhielt 1152 g eines Saat-Perlpolymerisates mit einer mittleren Teilchengröße von 365 $\mu$m, enger Teilchengrößenverteilung und glatter Oberfläche.

### Vergleichsbeispiel 1 (nicht erfindungsgemäß)

*VIa) Herstellung eines Perlpolymerisates ohne Comonomer*

In einem 4 L - Rührreaktor mit Gitterrührer, Kühler, Temperaturfühler sowie Thermostat und Temperaturschreiber wurde eine wässrige Vorlage aus 1443 g entionisiertem Wasser und 5,88 g Dinatriumhydrogenphosphat Dodecahydrat erzeugt. Zu dieser Vorlage wurde unter Rühren mit 200 U/min 864,9 g Saatpolymerisat aus Beispiel 1 gegeben.
Innerhalb von 30 Min wurde ein Gemisch aus 625,7 g Styrol, 109,5 g Divinylbenzol (81,3%ig) und 5,88 g Dibenzoylperoxid zugesetzt. Zur Entfernung von Luftsauerstoff wurde nun 15 Minuten mit Stickstoff begast. Anschließend wurde der Reaktorinhalt innerhalb 30 Minuten auf 30°C gebracht und weitere 30 Minuten bei dieser Temperatur gehalten. Es wurde dann eine Lösung von 3,2 g Methylhydroxyethylcellulose gelöst in 157 g Wasser zugesetzt und nochmals eine Stunde bei 30°C gerührt. Es wurde 16 Stunden auf 62°C und anschließend 2 Stunden auf 95°C erhitzt. Der Ansatz wurde nach dem Abkühlen über ein 315 $\mu$m-Sieb gewaschen und getrocknet. Man erhielt 1465 g eines monodispersen Perlpolymerisats mit einer mittleren Teilchengröße von 448 $\mu$m.

*V1b) Herstellung eines Kationenaustauschers*

In einem 4 L - Rührreaktor mit Gitterrührer, Temperaturfühler, Destillationsbrücke sowie Thermostat und Temperaturschreiber wurden 741 ml 87,8 gew.-%ige Schwefelsäure bei Raumtemperatur vorgelegt. Innerhalb 30 Minuten wurden 350 g Perlpolymerisat aus V1a) und 88 ml 1,2-Dichlorethan unter Rühren eingetragen. Der Reaktorinhalt wurde 30 Minuten bei 40 °C gerührt. Anschließend wurden innerhalb einer Stunde 159 ml Oleum (65 Gew.-%iges $SO_3$ in 100

gew.-%iger Schwefelsäure) zugegeben, ohne dass die Reaktortemperatur 90 °C überschreitet wurde. Danach wurde auf 115 °C aufgeheizt und 5 Stunden bei 115°C gerührt, wobei das 1,2-Dichlorethan über eine Destillationsbrücke abgetrennt wurde. Das Reaktionsgemisch wurde anschließend auf 140 °C gebracht und noch 3 Stunden bei 140 °C gerührt. Nach dem Abkühlen wurde die Suspension in eine Glassäule überführt. Schwefelsäure abnehmender Konzentration, beginnend mit 90 gew%-ig und endend mit reinem Wasser, wurden von oben auf die Säule aufgegeben. Man erhielt 1460 ml monodispersen Kationenaustauscher in der H-Form mit einer Totalkapazität, bezogen auf die H-Form, von 2,08 Eq/L.

Vergleichsbeispiel 2 (nicht erfindungsgemäß)

*V2a) Herstellung eines Perlpolymerisates ohne Comonomer*

In einem 4 L - Rührreaktor mit Gitterrührer, Kühler, Temperaturfühler sowie Thermostat und Temperaturschreiber wurde eine wässrige Vorlage aus 1342 g entionisiertem Wasser, 5,3 g Borsäure und 2,97 g 50 gew.%-ige Natronlauge erzeugt. Zu dieser Vorlage wurde unter Rühren mit 200 U/min 802,4 g Saatpolymerisat, hergestellt gemäß Beispiel 1, gegeben.
Innerhalb von 30 Min wurde ein Gemisch aus 687,7 g Styrol, 114,7 g Divinylbenzol (80,5%ig) und 2,57 g tert.-Butylperoxy-2-ethylhexanoat zugesetzt. Zur Entfernung von Luftsauerstoff wurde nun 15 Minuten mit Stickstoff begast. Anschließend wurde der Reaktorinhalt innerhalb 30 Minuten auf 30 °C gebracht und weitere 2 Stunden bei dieser Temperatur gehalten. Es wurde dann eine Lösung von 3,2 g Methylhydroxyethylcellulose gelöst in 157 g Wasser zugesetzt und nochmals eine Stunde bei 30 °C gerührt. Es wurde 11 Stunden auf 65°C und anschließend 2 Stunden auf 95°C erhitzt. Der Ansatz wurde nach dem Abkühlen über ein 315 5 $\mu$m-Sieb gewaschen und getrocknet. Man erhielt 1544 g eines monodispersen Perlpolymerisats mit einer mittleren Teilchengröße von 460 $\mu$m.

*V2b) Herstellung eines Kationenaustauschers*

In einem 4 L - Rührreaktor mit Gitterrührer, Temperaturfühler, Destillationsbrücke sowie Thermostat und Temperaturschreiber wurden 1400 ml 98 gew.-%ige Schwefelsäure vorgelegt und auf 100°C erhitzt. In 30 Minuten wurden 350 g Perlpolymerisat aus V2a) in 10 Portionen unter Rühren eingetragen. Anschließend wurde 30 Minuten bei 100 °C und 5 Stunden bei 115°C gerührt. Nach dem Abkühlen wurde die Suspension in eine Glassäule überführt. Schwefelsäure abnehmender Konzentration, beginnend mit 90 gew% ig und endend mit reinem Wasser, wurden von oben auf die Säule aufgegeben.
Man erhielt 1440 ml monodispersen Kationenaustauscher in der H-Form mit einer Totalkapazität, bezogen auf die H-Form, von 2,2 Eq/L.

**Vergleichsbeispiel 3 (nicht erfindungsgemäß)**

*V3a) Herstellung eines Perlpolymerisates mit Acrylnitril gemäß DE-A 10 122 896*

Es wurde wie in Beispiel V2a) verfahren, wobei 4,71 g Borsäure und 2,64 g 50 gew.%-ige Natronlauge in der Vorlage eingesetzt wurden. Hierzu wurden 891,5 g Saatpolymerisat, hergestellt wie in Beispiel 1, zugegeben, und ein Gemisch aus 560,3 g Styrol, 88,7 g Divinylbenzol (81,3%ig), 64,2 g Acrylnitril und 2,28 g tert.-Butylperoxy-2-ethylhexanoat zugesetzt. Es wurde 11 Stunden auf 61°C und anschließend 2 Stunden auf 130°C erhitzt.
Man erhielt 1505 g eines monodispersen Perlpolymerisats mit einer mittleren Teilchengröße von 442 $\mu$m.

*V3b) Herstellung eines Kationenaustauschers*

Es wurde wie im Beispiel V2b) verfahren, wobei 2800 ml 98 gew.-%ige Schwefelsäure vorgelegt wurden und 700 g Perlpolymerisat aus V3a) eingetragen und 5 Stunden bei 105°C gerührt wurden.
Man erhielt 3000 ml monodispersen Kationenaustauscher in der H-Form mit einer Totalkapazität, bezogen auf die H-Form, von 1,92 Eq/L.

**Beispiel 2 (erfindungsgemäß)**

*2a) Herstellung eines Perlpolymerisates mit Diethylenglykoldivinylether*

In einem 4 L - Rührreaktor mit Gitterrührer, Kühler, Temperaturfühler sowie Thermostat und Temperaturschreiber wurde eine wässrige Vorlage aus 1443 g entionisiertem Wasser und 5,88 g Dinatriumhydrogenphosphat Dodecahydrat erzeugt. Zu dieser Vorlage wurde unter Rühren mit 200 U/min 864,9 g Saatpolymerisat, hergestellt wie in Beispiel 1, gegeben.

Innerhalb von 30 Min wurde ein Gemisch aus 625,7 g Styrol, 109,5 g Divinylbenzol (81,4%ig) und 5,88 g Dibenzoylperoxid zugesetzt. Zur Entfernung von Luftsauerstoff wurde nun 15 Minuten mit Stickstoff begast. Anschließend wurde der Reaktorinhalt innerhalb 30 Minuten auf 30 °C gebracht und 30 Minuten bei dieser Temperatur gehalten. Es wurde dann eine Lösung von 3,2 g Methylhydroxyethylcellulose gelöst in 157 g Wasser zugesetzt und nochmals 1 Stunde bei 30 °C gerührt. Es wurde 16 Stunden auf 62°C erhitzt. Dann wurden bei 62 °C in 2 getrennten Zuläufen innerhalb 30 Minuten 30 g Diethylenglykoldivinylether sowie eine Lösung von 5 g Kaliumperoxodisulfat in 50 ml Wasser zugegeben. Anschließend wurde 2 Stunden auf 95°C erhitzt. Der Ansatz wurde nach dem Abkühlen über ein 315 $\mu$m-Sieb gewaschen und getrocknet. Man erhielt 1539 g eines monodispersen Perlpolymerisats mit einer mittleren Teilchengröße von 448 $\mu$m

*2b) Herstellung eines Kationenaustauschers*

Es wurde wie in Beispiel V3b) verfahren, wobei 700 g Perlpolymerisat aus 2a) eingetragen wurden und statt 5 Stunden bei 105 °C, 10 Stunden bei 115°C gerührt wurden.

Man erhielt 3920 ml monodispersen Kationenaustauscher in der H-Form mit einer Totalkapazität, bezogen auf die H-Form, von 2,12 Eq/L.

**Beispiel 3 (erfindungsgemäß)**

*3a) Herstellung von Perlpolymerisaten mit Diethylenglykoldivinylether*

In einem 4 L - Rührreaktor mit Gitterrührer, Kühler, Temperaturfühler sowie Thermostat und Temperaturschreiber wurde eine wässrige Vorlage aus 1443 g entionisiertem Wasser, 4,85 g Borsäure und 2,72 g 50 gew.%-ige Natronlauge erzeugt. Zu dieser Vorlage wurde unter Rühren mit 200 U/min 864,9 g Saatpolymerisat, hergestellt wie in Beispiel 1, gegeben.

Innerhalb von 30 Min wurde ein Gemisch aus S g Styrol (siehe Tabelle), 109,5 g Divinylbenzol (81,3%ig), X g Diethylenglykoldivinylether (DEGDVE, siehe Tabelle) und 2,35 g tert.-Butylperoxy-2-ethylhexanoat zugesetzt. Zur Entfernung von Luftsauerstoff wurde nun 15 Minuten mit Stickstoff begast. Anschließend wurde der Reaktorinhalt innerhalb von 30 Minuten auf 30 °C gebracht und 30 Minuten bei dieser Temperatur gehalten. Es wurde dann eine Lösung von 3,2 g Methylhydroxyethylcellulose gelöst in 157 g Wasser zugesetzt und nochmals eine Stunde bei 30 °C gerührt. Es wurde 11 Stunden auf 65°C und anschließend 2 Stunden auf 95°C erhitzt. Der Ansatz wurde nach dem Abkühlen über ein 315 $\mu$m-Sieb gewaschen und getrocknet. Man erhielt Y g eines monodispersen Perlpolymerisats (siehe Tabelle).

*3b) Herstellung von Kationenaustauschern*

Es wurde wie im Beispiel V2b) verfahren, wobei jeweils 350 g Perlpolymerisat aus 3a) eingetragen wurden.

Man erhielt Z ml monodisperse Kationenaustauscher in der H-Form mit einer Totalkapazität, bezogen auf die H-Form, von TK Eq/L (siehe Tab. 1).

Tab. 1

| Beispiel | Menge S an Styrol | Menge X an DEGDVE | Ausbeute Y an Polymer | Ausbeute Z an Harz | TK des Harzes |
|---|---|---|---|---|---|
| 3.1 | 618,3 g | 7,4 g | 1575 g | 1400 ml | 2,20 Eq/L |
| 3.2 | 603,5 g | 22,2 g | 1574 g | 1400 ml | 2,18 Eq/L |
| 3.3 | 588,7 g | 37,0 g | 1580 g | 1380 ml | 2,17 Eq/L |

**Beispiel 4 (erfindungsgemäß)**

*4a) Herstellung eines Perlpolymerisates mit Butandioldivinylether*

Es wurde wie in Beispiel 2a verfahren, wobei 30 g Butandioldivinylether anstelle von 30 g Ethylenglykoldivinylether eingesetzt wurden.

Man erhielt 1550 g eines monodispersen Perlpolymerisats.

*4b) Herstellung eines Kationenaustauschers*

In einem 4 L - Rührreaktor mit Gitterrührer, Temperaturfühler, Destillationsbrücke sowie Thermostat und Temperaturschreiber wurden 1200 ml 98 gew.-%ige Schwefelsäure vorgelegt und auf 100°C erhitzt. In 30 Minuten wurden 300 g Perlpolymerisat aus 4a) in 10 Portionen unter Rühren eingetragen. Anschließend wurde 5 Stunden bei 115°C und 2 Stunden bei 135 °C gerührt. Nach dem Abkühlen wurde die Suspension in eine Glassäule überführt. Schwefelsäure abnehmender Konzentration, beginnend mit 90 gew%ig und endend mit reinem Wasser, wurde von oben auf die Säule aufgegeben.
Man erhielt 1280 ml monodispersen Kationenaustauscher in der H-Form mit einer Totalkapazität, bezogen auf die H-Form, von 2,19 Eq/L.

**Beispiel 5 (erfindungsgemäß)**

*5a) Herstellung eines Perlpolymerisates mit Ethylenglykolmonovinylether*

Es wurde wie in Beispiel 2a verfahren, wobei 30 g Ethylenglykolmonovinylether anstelle von 30 g Ethylenglykoldivinylether eingesetzt wurden.
Man erhielt 1670 g eines monodispersen Perlpolymerisats.

*5b) Herstellung eines Kationenaustauschers*

Es wurde wie im Beispiel 4b) verfahren, wobei 1400 ml 98 gew.-%ige Schwefelsäure vorgelegt und 350 g Perlpolymerisat aus 5a) eingetragen wurden.
Man erhielt 1470 ml monodispersen Kationenaustauscher in der H-Form mit einer Totalkapazität, bezogen auf die H-Form, von 2,21 Eq/L.

**Beispiel 6 (erfindungsgemäß)**

*6a) Herstellung eines Perlpolymerisates mit Vinylacetat*

Es wurde wie in Beispiel 2a verfahren, wobei 30 g Vinylacetat anstelle von 30 g Ethylenglykoldivinylether eingesetzt wurden.
Man erhielt 1264 g eines monodispersen Perlpolymerisats.

*6b) Herstellung eines Kationenaustauschers*

Es wurde wie in Beispiel 5b mit 350 g Perlpolymerisat aus 6a) verfahren.
Man erhielt 1480 ml monodispersen Kationenaustauscher in der H-Form mit einer Totalkapazität, bezogen auf die H-Form, von 2,17 Eq/L.

**Beispiel 7 (erfindungsgemäß)**

*7a) Herstellung eines Perlpolymerisates mit Diethylenglykodivinylether*

Das Beispiel 2a wurde wiederholt. Man erhielt 1570 g eines monodispersen Perlpolymerisats.

*7b) Herstellung eines Kationenaustauschers*

Es wurde analog Vergleichbeispiel V1b) verfahren, wobei 664 ml 84,7 gew.-%ige Schwefelsäure bei Raumtemperatur vorgelegt wurden und 350 g Perlpolymerisat aus 7a) eingesetzt und 227 ml Oleum zugegeben wurden.
Man erhielt 1500 ml monodispersen Kationenaustauscher in der H-Form mit einer Totalkapazität, bezogen auf die H-Form, von 2,08 Eq/L.

**Beispiel 8 (erfindungsgemäß)**

*8a) Herstellung eines Perlpolymerisates mit Diethylenglykoldivinylether*

In einem 4 L - Rührreaktor mit Gitterrührer, Kühler, Temperaturfühler sowie Thermostat und Temperaturschreiber wurde eine wässrige Vorlage aus 1400 g entionisiertem Wasser, 6,88 g Dinatriumhydrogenphosphat Dodekahydrat und 90,3 g einer 2%igen wässrigen Lösung von Methylhydroxyethylcellulose erzeugt.

Danach wurde ein Gemisch aus 1316,4 g Styrol, 137,7 g Divinylbenzol (80,3%ig), 14,7 g Diethylenglykoldivinylether und 11,8 g Dibenzoylperoxid (75 Gew.-% in Wasser) zugesetzt. Der Reaktorinhalt wurde 30 Minuten bei Raumtemperatur stehen lassen, wobei sich 2 Phasen bildeten. Dann wurde der Rührer mit 180 U/Min eingeschaltet und es wurde 30 Minuten bei Raumtemperatur gerührt. Der Reaktorinhalt wurde anschließend 16 Stunden bei 62 °C und 2 Stunden bei 95 °C erhitzt. Der Ansatz wurde nach dem Abkühlen über ein 315 μm-Sieb gewaschen und getrocknet. Man erhielt 1441 g eines heterodispersen Perlpolymerisats.

*8b) Herstellung eines Kationenaustauschers*

Es wurde analog Vergleichbeispiel V1b) verfahren, wobei 1110 ml 86 gew.-%ige Schwefelsäure bei Raumtemperatur vorgelegt, 350 g Perlpolymerisat aus 8a) eingesetzt und 322 ml Oleum zugegeben wurden. Es wurde lediglich 5 Stunden bei 115 °C gerührt (ohne 140 °C-Stufe).

Man erhielt 1590 ml heterodispersen Kationenaustauscher in der H-Form mit einer Totalkapazität, bezogen auf die H-Form, von 1,90 Eq/L.

**Beispiel 9 (erfindungsgemäß)**

*9a) Herstellung eines makroporösen Perlpolymerisates mit Diethylenglykoldivinylether*

In einem 4 L - Rührreaktor mit Gitterrührer, Kühler, Temperaturfühler sowie Thermostat und Temperaturschreiber wurde eine wässrige Vorlage aus 1112 g entionisiertem Wasser, 7,86 g Dinatriumhydrogenphosphat Dodekahydrat und 149 g einer 2,2%igen wässrigen Lösung von Methylhydroxyethylcellulose erzeugt.

Danach wurde ein Gemisch aus 812 g Styrol, 140,2 g Divinylbenzol (81,8%ig), 19,1 g Diethylenglykoldivinylether, 421 g Isododekan und 5,73 g tert.-Butylperoxy-2-ethylhexanoat zugesetzt. Der Reaktorinhalt wurde 20 Minuten bei Raumtemperatur stehen lassen, wobei sich 2 Phasen bildeten. Dann wurde der Rührer mit 300 U/Min eingeschaltet und es wurde 30 Minuten bei Raumtemperatur gerührt. Der Reaktorinhalt wurde anschließend 7 Stunden bei 70 °C und 2 Stunden bei 95 °C erhitzt. Der Ansatz wurde nach dem Abkühlen über ein 315 μm-Sieb gewaschen und getrocknet. Man erhielt 959 g eines makroporösen, heterodispersen Perlpolymerisats (U = Umdrehungen).

*9b) Herstellung eines makroporösen Kationenaustauschers*

Es wurde analog Vergleichbeispiel V2b) verfahren, wobei 350 g Perlpolymerisat aus 9a) bei 115°C (statt bei 100°C) in die Schwefelsäure eingetragen wurden.

Man erhielt 1600 ml heterodispersen Kationenaustauscher in der H-Form mit einer Totalkapazität, bezogen auf die H-Form, von 1,90 Eq/L.

**Vergleichsbeispiel 4 (nicht erfindungsgemäß)**

*V4a) Herstellung eines makroporösen Perlpolymerisates ohne Comonomer*

Es wurde wie im Beispiel 9a verfahren, wobei das Diethylenglykoldivinylether weggelassen wurde. Man erhielt 936 g eines makroporösen, heterodispersen Perlpolymerisats.

*V4b) Herstellung eines makroporösen Kationenaustauschers*

Es wurde wie im Beispiel 9b) verfahren, wobei 350 g Perlpolymerisat aus V4a) bei 115°C in die Schwefelsäure eingetragen wurden.

Man erhielt 1640 ml heterodispersen Kationenaustauscher in der H-Form mit einer Totalkapazität, bezogen auf die H-Form, von 1,87 Eq/L.

Tab. 2: Messergebnisse zur osmotischen und Oxidations-Stabilität der Beispiele 2 bis 9 sowie der Vergleichbeispiele 1 bis 4

| Beispiel | TK (Eq/L) | OS | QS | Rotfärbungstest nach 4 Wochen | | |
|----------|-----------|-----|------|------|------------|------|
| | | | | pH | Abs. 225 nm | Note |
| V1 | 2,08 | 90 | 84 | 3,33 | 2,91 | 1 |
| V2 | 2,20 | 97 | n.b. | 3,38 | 2,10 | 2 |
| V3 | 1,92 | 97 | 89 | 2,79 | >4 | 3 |
| V4 | 1,87 | 100 | n.b. | 3,46 | 2,57 | 2 |
| 2 | 2,12 | 95 | 93 | 3,64 | 0,59 | 1 |
| 3.1 | 2,20 | 94 | 92 | 3,55 | 1,19 | 1 |
| 3.2 | 2,18 | 98 | 91 | 3,50 | 0,95 | 1 |
| 3.3 | 2,17 | 96 | 95 | 3,62 | 0,61 | 0 |
| 4 | 2,19 | 97 | 97 | 3,57 | 0,61 | 1 |
| 5 | 2,21 | 99 | 96 | 3,54 | 1,22 | 1 |
| 6 | 2,17 | 98 | 90 | 3,54 | 1,04 | 2 |
| 7 | 2,08 | 99 | 85 | 3,58 | 0,70 | 1 |
| 8 | 1,90 | 95 | n.b. | 3,56 | 2,00 | 2 |
| 9 | 1,90 | 100 | n.b. | 3,61 | 1,64 | 3 |
| TK = Totalkapazität; OS = Originalstabilität; QS = Quellungsstabilität | | | | | | |

**Beispiel 10**

*Bestimmung der Oxidationsstabilität der Harze*

Die Harze aus den Beispielen 2 bis 8 sowie aus den Vergleichsbeispielen V1 bis V3 wurden dem Rotfärbungstest unterzogen.

Der pH-Wert gibt Auskunft darüber, wie viel lösliche Säure (hauptsächlich Polystyrolsulfonsäure, sogennantes Leaching, kombiniert mit geringen Mengen an Schwefelsäure aus der Hydrolyse der Sulfonsäuregruppen des Harzes) nach einer gegebenen Lagerzeit an der Luft gebildet wurde; die Absorption bei 225 nm ist ein Maß für die abgegebenen, wasserlöslichen aromatischen Verbindungen, in diesem Fall oligomere und polymere Styrolsulfonsäuren.

Bei dem Test gilt:

- Um so höher der pH-Wert des Eluats, desto weniger lösliche Säure wurde vom Harz abgegeben, desto höher ist die Oxidationsstabilität des Harzes;

- um so niedriger der Absorptionswert des Eluats bei 225 nm, desto weniger lösliche aromatische Verbindungen wurden vom Harz abgegeben, desto höher ist die Oxidationsstabilität des Harzes;

- um so niedriger die Note bei der visuellen Beurteilung des Eluats, desto farbloser das Eluat.

Die Ergebnisse der osmotischen Stabilität und des Oxidationsstabilitätstests für die verschiedenen Beispiele und Vergleichbeispiele werden in der Tab. 2 angegeben.

Aus der Tab. 2 ist zu erkennen, dass die erfindungsgemäßen stark sauren Kationenaustauscher Totale Kapazitäten besitzen, die der Totalen Kapazität der Vergleichbeispiele in nichts nachsteht.

Man erkennt, dass die erfindungsgemäßen stark sauren Kationenaustauscher sehr hohe Original- und Quellungsstabilitätswerte aufweisen, die vergleichbar oder höher sind, als die Werte der dem Stand der Technik entsprechenden Vergleichsbeispielen.

Alle erfindungsgemäßen Kationenaustauscher weisen im Rotfärbungstest einen höheren pH-Wert des Eluats und einen niedrigeren Absorptionswert des Eluats bei 225 nm als die Vergleichbeispiele auf. Dies belegt eine deutlich reduzierte Abgabe von löslichen Polystyrolsulfonsäuren aus den erfindungsgemäßen Harzen und somit die höhere Oxidationsstabilität der erfindungsgemäßen Harze.

Tab. 2 ist auch zu entnehmen, dass die Verbesserung des Eigenschaftsprofils der erfindungsgemäßen stark sauren Kationenaustauscher für die Divinyletherverbindungen besonders ausgeprägt ist (Beispiele 2,3,4,7).

Durch Vergleich der Beispiele 2, 3 und 4 sieht man, dass der Effekt der Zugabe von Vinylether und/oder Vinylester bei der Polymerisation unabhängig von der Art und vom Zeitpunkt des Comonomereinbaus ist.

Am Beispiel 9, verglichen mit Vergleichsbeispiel 4 wird ersichtlich, dass die Verbesserung der Oxidationsstabilität der starksaurem Kationenaustauscher durch den erfindungsgemäßen Einbau von Vinylether(n) und/oder Vinylester (n) auch bei makroporösen Harzen eintritt.

## Patentansprüche

1. Starksaure Kationenaustauscher, **dadurch gekennzeichnet, dass** sie durch Sulfonierung von Perlpolymerisaten aus vinylaromatischen Monomeren, Vernetzern und 0,2 bis 20 Gew.-% Vinylester gewonnen werden.

2. Starksaure Kationenaustauscher gemäß Anspruch 1, **dadurch gekennzeichnet, dass** die Perlpolymerisate Copolymerisate aus mindestens einem monoethylenisch ungesättigten, aromatischen Monomer, mindestens einem Vernetzer und mindestens einem Vinylester sind.

3. Starksaure Kationenaustauscher nach Anspruch 1 oder 2, **dadurch gekennzeichnet, dass** diese eine monodisperse Teilchengrößenverteilung aufweisen, weshalb sie einen Volumenanteil an Teilchen zwischen 0,9 D und 1,1 D von mindestens 75 Vol.-% aufweisen worin D die mittlere Teilchengröße bedeutet.

4. Starksaure Kationenaustauscher nach einem der Ansprüche 1 bis 3, **dadurch gekennzeichnet, dass** sie aus Perlpolymerisaten gewonnen werden, die nach dem Seed-Feed-Verfahren hergestellt wurden.

5. Starksaure Kationenaustauscher gemäß der Ansprüche 1 bis 4, **dadurch gekennzeichnet, dass** deren Monomeren Porogene zugesetzt werden.

6. Verfahren zur Herstellung starksaurer Kationenaustauscher gemäß Anspruch 1, **dadurch gekennzeichnet, dass** man:

   a) monodisperse oder heterodisperse Perlpolymerisate aus vinylaromatischen Monomeren, Vernetzern und 0,2 bis 20 Gew.-% Vinylester durch Suspensionspolymerisation herstellt und
   b) diese Perlpolymerisate durch Einwirkung von Schwefelsäure, Schwefeltrioxid und/oder Chlorsulfonsäure in starksaure Kationenaustauscher überführt.

7. Verwendung der starksauren Kationenaustauscher gemäß der Ansprüche 1 bis 5 bei der Trinkwasseraufbereitung, bei der Herstellung von Kraftwasser und Reinstwaser, zur chromatographischen Trennung von Glucose und Fructose und als Katalysatoren für verschiedene chemische Reaktionen.

8. Verwendung gemäß Anspruch 7, **dadurch gekennzeichnet, dass** die starksauren Kationenaustauscher als Katalysatoren bei der Bisphenol-A-Herstellung aus Phenol und Aceton eingesetzt werden.

Europäisches Patentamt
European Patent Office
Office européen des brevets

## EUROPÄISCHER RECHERCHENBERICHT

**Nummer der Anmeldung**

EP 11 15 5485

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 692 32 489 T2 (ARCH DEV CORP [US]; UNIV TENNESSEE RES CORP [US]) 26. September 2002 (2002-09-26) | 1,2,5-7 | INV. B01J39/20 B01J39/26 C08F8/36 C02F1/42 B01J31/10 C08F257/02 |
| Y | * Seite 14, letzter Absatz - Seite 15, Absatz 5 * * Seite 17; Beispiel 4 * * Seite 24; Abbildung 1 * * Seite 25; Abbildung 1 * * Anspruch 3 * ----- | 3,4,8 | |
| Y,D | EP 1 000 659 A1 (BAYER AG [DE] BAYER CHEMICALS AG [DE]) 17. Mai 2000 (2000-05-17) | 3,4,8 | |
| A | * Zusammenfassung * * Absatz [0006] * * Absatz [0010] * ----- | 1,2,5-7 | |

**RECHERCHIERTE SACHGEBIETE (IPC)**

B01J
C08F

Der vorliegende Recherchenbericht wurde für alle Patentansprüche erstellt

| Recherchenort | Abschlußdatum der Recherche | Prüfer |
|---|---|---|
| Den Haag | 30. Mai 2011 | Klemps, Christian |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 15 5485

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-05-2011

| Im Recherchenbericht angeführtes Patentdokument | Datum der Veröffentlichung | Mitglied(er) der Patentfamilie | | Datum der Veröffentlichung |
|---|---|---|---|---|
| DE 69232489 T2 | 26-09-2002 | AT | 214303 T | 15-03-2002 |
| | | CA | 2126222 A1 | 08-07-1993 |
| | | DE | 69232489 D1 | 18-04-2002 |
| | | EP | 0618843 A1 | 12-10-1994 |
| | | JP | 7508452 T | 21-09-1995 |
| | | JP | 3345008 B2 | 18-11-2002 |
| | | WO | 9312882 A1 | 08-07-1993 |
| | | US | 5449462 A | 12-09-1995 |
| | | US | 5281631 A | 25-01-1994 |
| EP 1000659 A1 | 17-05-2000 | DE | 19852667 A1 | 18-05-2000 |
| | | HU | 9904255 A2 | 28-10-2000 |
| | | JP | 2000140652 A | 23-05-2000 |
| | | KR | 20000047635 A | 25-07-2000 |
| | | TW | I229012 B | 11-03-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0101943 A2
- EP 1000659 A
- DE 100122896 A
- US 4500652 A
- DE 3230559 A
- EP 868444 A
- EP 0366258 A
- EP 0046535 A
- EP 0617714 A

**In der Beschreibung aufgeführte Nicht-Patentliteratur**

- *Pure Appl. Chem.,* 2004, vol. 76 (4), 889-906
- *IUPAC recommendations,* 2003
- **ANTHONY C. BEVILACQUA.** Advances in Resistivity Instrumentation for UPW Systems of the Future. *1998 Semiconductor Pure Water and Chemicals Conference,* 02. Marz 1998